# EUROPEAN PATENT SPECIFICATION

(11) **EP 2 717 931 B1**
(45) Date of publication and mention of the grant of the patent: **07.08.2019**
(21) Application number: 12796310.6
(22) Date of filing: 04.06.2012
(51) Int. Cl.: A61L 15/22, A61F 13/15, A61F 13/02, A61K 9/70, A61L 15/58

(54) **DRESSINGS AND RELATED METHODS THEREFOR**
VERBÄNDE UND ZUGEHÖRIGE VERFAHREN
PANSEMENTS ET PROCÉDÉS ASSOCIÉS POUR CEUX-CI

(30) Priority: 06.06.2011 US 201113153842
(43) Date of publication of application: 16.04.2014
(73) Proprietor: KPR U.S., LLC, Mansfield, MA 02048 (US)
(72) Inventor: MOGHE, Ajit, K., Bellingham, MA 02019 (US); GAHAN, Richard, E., Wrentham, MA 02093 (US); PATEL, Harish, A., Norfolk, MA 02056 (US)
(74) Representative: Prock, Thomas
(86) International application number: PCT/US2012/040697
(87) International publication number: WO 2012/170334

(56) References cited:
- EP-B1- 0 625 914
- US-A- 5 318 552
- US-A- 5 505 719
- US-A- 5 667 864
- US-A1- 2005 054 998
- US-A1- 2007 078 365
- US-A1- 2008 167 594
- US-A1- 2010 055 158
- US-A1- 2010 106 122
- US-A1- 2010 260 824
- US-A1- 2010 260 824

## Description

### BACKGROUND

### Technical Field

The present disclosure generally relates to dressings, and more particularly, relates to wound dressings and adhesive bandages that include a non-sticking film having a first polyester with a first melting point or melting range and a second polyester with a second melting point or melting range that is lower than the first melting point or melting range.

### Description of Related Art

Karami et al., in U.S. Patent No. 5,167,613, disclosed a composite vented wound dressing with a primary dressing placement on the skin to cover a wound and an overlaying secondary dressing containing an absorbent pad for receiving and retaining wound fluids diffusing thereto from the wound.

Patel, in U.S. Patent No. 5,465,735, disclosed a wound dressing with an absorbent pad for receiving and retaining wound fluids sandwiched between first and second outer sheet materials. The first sheet material for placement on the wound can be a perforated non-adherent film for preventing the dressing from sticking to the wound. The second sheet material can be characterized as being bacteria-impermeable.

Patel, in U.S. Patent No. 5,632,731, disclosed a non-adherent wound dressing with a multilayer absorbent pad comprising an inner layer of a low density absorbent material for receiving fluids diffusing from the wound and an overlying layer of a high density absorbent material for receiving and retaining wound fluids diffusing through the inner layer.

Landoll, in US Patent No. 5,667,864, disclosed absorbent laminates and methods of making the same.

### SUMMARY

One or more aspects of the invention can be directed to a dressing for applying on a wound. In accordance with one or more embodiments of such aspects of the invention, the dressing comprises an absorbent pad having a wound-facing surface and a second surface opposite from the wound-facing surface, and a non-stick film secured to the absorbent pad on the wound-facing surface, the non-stick film having a first region consisting essentially of a first polyester and a second region consisting essentially of a second polyester having a lower melting point than the first polyester. The second region can define a securing layer attaching, or is attachable to, the non-stick film to the wound-facing surface of the absorbent pad. The dressing can further comprise a second film secured to the absorbent pad on the second surface thereof. The second film can have a third region comprising the first polymeric material and a fourth region comprising the second polymeric material. In accordance with further variants of such embodiments, the fourth region can define a second securing layer attaching, or is attachable to, the second film to the second surface of the absorbent pad. The second polyester can comprise an amorphous polyester having a melting temperature range in a range of from 120°C to 200°C. The first polyester can comprise or consist essentially of polyethylene terephthalate with a melting temperature range in a range of from 200°C to 275°C. The second polyester can comprise or consist essentially of polyethylene terephthalate with a glass transition temperature in a range of from 60°C to 88°C. The dressing can further comprise a backing substrate having an adhesive material on a surface thereof. In accordance with a variant of such one or more embodiments, the backing substrate can be secured to the absorbent pad at the second surface. The backing substrate can comprise or consist essentially of a woven fabric and the adhesive material can comprise or consist essentially of an acrylic. The non-stick film can have one or more perforated regions, each of which can have a plurality of perforations extending through the thickness of the film. The absorbent pad can comprise a fiber selected from the group consisting of cellulosic fibers such as viscose, cotton, fiberized paper pulp, and lyocell, and polymeric fibers such as polypropylene, and polyester fibers, as well as combinations thereof. The absorbent pad can have a first, absorbent layer comprising, consisting essentially, or consisting of a low density absorbent fabric and a second, wicking layer comprising, consisting essentially of, or consisting of a high density absorbent fabric. The dressing can further comprise at least one therapeutic agent in any of the absorbent pad and the non-stick film. In one or more variants of any one or more embodiments of the invention, the absorbent pad can comprise polyhexamethylene biguanide (PHMB).

One or more aspects of the invention can be directed to a method of producing a dressing. The method of producing the dressing can comprise securing a non-stick film to an absorbent pad at a wound-facing surface thereof, wherein the non-stick film comprises a first region consisting essentially of a first polyester and a second region consisting essentially of a second polyester, the second polyester having a lower melting point that the first polyester. Securing the non-stick film to the absorbent pad can comprise enclosing the absorbent pad within an envelope comprising the non-stick film. Securing the non-stick film to the absorbent pad can comprise melting at least a portion of the second polyester. Securing the non-stick film to the absorbent pad can comprise exposing the non-stick film to an environment with a temperature in a range of from 140°C to 200°C. The method of producing the dressing can further comprise attaching the absorbent pad to a backing substrate having an adhesive material on a surface thereof. The first polyester can have a melting range in a range of from 200°C to 275°C, and, in accordance with further embodiments of the invention, the second polyester can have a melting range in a range of from 120°C to 200°C. The absorbent pad can have a first, absorbent layer comprising or consisting essentially of a low density absorbent fabric and a second, wicking layer comprising or consisting of a high density absorbent fabric. Securing the non-stick film to the absorbent pad can comprise securing the second region of the non-stick film to the second wicking layer of the absorbent pad. The method can further comprise introducing at least one therapeutic agent in any of the absorbent pad and the non-stick film. Introducing the at least one therapeutic agent can comprise immersing at least a portion of the absorbent pad in a fluid comprising polyhexamethylene biguanide to produce a PHMB-immersed pad; and drying the PHMB-immersed pad to produce a PHMB-containing pad. In one or more embodiments of the invention, securing the non-stick film to the absorbent pad can comprise securing the second region of the non-stick film to the PHMB-containing pad.

### BRIEF DESCRIPTION OF THE DRAWINGS

The present disclosure will be more clearly understood from the following description in connection with the accompanying drawings in which:
FIG. 1 is a plan view of an adhesive bandage, in accordance with at least one embodiment described herein;
FIG. 2 is an elevational view of the adhesive bandage shown in FIG. 1;
FIG. 3 is a plan view of a dressing, in accordance with at least one embodiment described herein;
FIG. 4 is an elevational view of the dressing, shown in FIG. 3, and further showing an absorbent pad between a non-stick films and a second film;
FIG. 5 is a plan view of an adhesive bandage, in accordance with at least one embodiment described herein; and
FIG. 6 is an elevational view of the adhesive bandage shown in FIG. 5.

### DETAILED DESCRIPTION

One or more aspects of the invention can be directed to dressings and bandages that are typically applied to any of cuts, abrasions, and wounds to collect blood and other wound exudates. One or more aspects of the invention can be directed to improving the adherence of a non-stick film or adherent resistant layer on an absorbent pad of dressings and bandages. One or more further aspects of the invention can be directed to increasing absorbance capacity for blood, wound exudates, or other fluids of dressings and bandages while reducing the likelihood of separation or delamination of the non-stick film from the absorbent pad. One or more still further aspects of the invention can be directed to providing a non-stick film, at a wound side of dressings and bandages, and an adherent surface or layer at the non-wound facing side of dressings and bandages. One or more yet further aspects of the invention can be directed to facilitating bonding a non-stick film to an absorbent pad of dressings and bandages, which can allow the dressing or bandage to withstand greater stresses associated with a bleeding and/or oozing wound.

In accordance with one or more embodiments of the invention, a dressing can comprise at least one absorbent pad having a bottom, or a first surface, attached to a backing material or substrate and a top, typically a surface nearest a wound, or facing a wound, and a non-stick film having non-wound facing side positioned next to the top, or secured to the first surface, of the absorbent pad, as well as a wound-facing side, intended to be against or facing the wound. The non-stick film comprises a first region consisting essentially of a first polyester and a second region consisting essentially of a second polyester. The first and second polyesters may be combined in a multilayer configuration such that the first and second polyesters may be combined to form a single or unitary film.

The first and second polyesters have differing melting points, melting ranges, softening points, or softening ranges. The second polyester has a lower melting point than the first polyester.

In accordance with some embodiments of the invention, the adhesive bandage can comprise a backing material or substrate, an adhesive applied to the backing material or substrate, an absorbent pad having a bottom attached to the backing material or substrate and a top nearest a wound, and, a non-stick film with a non-wound facing surface positioned next or secured to the top, or a first surface of the absorbent pad, and a wound-facing surface intended to face the wound during use. The non-stick film consists essentially of a first polyester having a first melting point, or a first melting temperature range, and a second polyester having a second melting point, or a first melting temperature range, such that the first melting point or range is greater than the second melting point or range. In other cases, at least a portion of the first melting temperature range is greater than at least a portion of the second melting temperature range.

In any of the various embodiments disclosed herein, the non-stick film can have a thickness in a range of from about 10 microns to about 50 microns. Typically, the non-stick film has a uniform thickness profile.

At least a portion of the non-stick film can have a perforated region having a plurality perforations or apertures through the thickness of the film. When utilized, the second film can have a perforated region with a plurality of perforations or apertures. The perforations can be uniformly distributed through the at least a portion of the non-stick film. In a variant of such embodiments, the density of the perforations can be varied. Typically, the perforated region has a perforation density in a range of from about 300 perforations per square inch to about 400 perforations per square inch. Each of the plurality of perforations can have any geometrical shape but each typically has circular profile. The size of any of the perforations can vary and can be in a range of from about 0.01 mm to 1 mm; typically, the perforations have a nominal diameter in a range of from about 0.5 mm to about 1 mm.

The non-stick film has a first region consisting essentially of a first polyester and a second region consisting essentially of a second polyester having a lower melting point than the first polyester. In some embodiments of the invention, the second polyester has a melting point or a melting range in a range of from 120°C to 200°C. In some further variant of some embodiments of the invention, the second polyester has a melting point or melting range in a range of from 200°C to 275 °C. Some non-limiting examples include polyesters, such as polytetrafluoroethylene and polyethylene terephthalate. In some embodiments of the invention, the first polyester comprises a polyethylene terephthalate, such as an amorphous polyethylene terephthalate having a melting point less than about 200°C and the second polyester comprises crystalline or at least partially crystalline polyethylene terephthalate having a higher melting point or melting range higher than that of the first polyester. In particularly useful embodiments of the invention, the non-stick film may include a first layer comprising a polyethylene terephthalate having a melting point of less than or equal to 200°C, and a second layer comprising a polyethylene terephthalate having a melting point in a range of from 200°C to 275°C.

In accordance with further aspects of the invention, the first region of the non-stick film can have a glass transition temperature that differs from the glass transition temperature of the second region of the non-stick film. For example, the non-stick film can have a region consisting essentially of a polyester with a glass transition temperature in a range of from 60°C to 88°C, and, in some cases, can further have another region consisting essentially of another polyester with another, higher glass transition temperature. In some particular embodiments of the invention, the non-stick film can consist essentially of an at least partially crystalline polyester and an at least partially amorphous polyester. For example, the first region can consist essentially of an at least partially crystalline polyester; and the second region can consist essentially of an at least partially amorphous polyester. In some configurations or variants thereof, non-stick film can have a decreasing crystallinity gradient from, for example, the first region to the second region. In some further configurations or variants thereof, non-stick film can have an increasing gradient of an amount or level of amorphous polyester from, for example, the second region to the first region.

In some particular embodiments of the dressings or bandages, the thickness of the amorphous layer can be at least about 25 microns, and can be in a range of from 25 microns to 100 microns.

In embodiments wherein the same polymeric material is used to form the non-stick films described herein, the melting point may be altered or tailored by utilizing polyesters with differing molecular weights. In embodiments, the difference in melting points between the first and second polyesters used to form the non-stick film may range from 1 to 100 degrees Celsius, from 5 to 50 degrees Celsius, in still other embodiments, from 10 to 25 degrees Celsius.

The non-stick film may be a multilayer film including at least two layers, wherein a layer, typically the layer closest to the absorbent pad, is made from a polyester having a lower melting point than another polymeric material in another layer that is closest to the wound. It is envisioned that the multilayer film may be exposed to heat suitable at a temperature that softens and/or melts at least a portion of the film thereby facilitating adherence of the non-stick film to the absorbent pad, while maintaining the non-stick film ability to not adhere to the wound during use. For example, the non-stick film can have a first layer consist essentially of an amorphous polyester having a melting point, melting temperature range, or softening point of at least about 200°C, in some cases, at least about 225°C, and, in some advantageous configurations, can have a melting temperature range in a range of from 200°C to 275°C. The second layer can consist essentially of a polyester having a melting point, melting temperature range, or softening point of less than about 225°C, in some cases, less than about 200°C, and, in some advantageous configurations, can have a melting temperature range in a range of from 120°C to 200°C. During production, any of the one or more non-stick films may thus adhered to any of the one or more absorbent pads by raising the temperature of at least a portion of the non-stick film to melting temperature conditions or environments, such as a temperature in a range of from 140°C to 200°C to at least partially melt or soften at least a portion of the second layer; followed by cooling to about room temperature to effect adherence of the non-stick film to the absorbent pad. Unlike adhesives, the first polymeric layer will remain after being combined with the absorbent pad. In accordance with some embodiments of the invention, the non-stick film, the absorbent pad, or both may be heated to effect at least melting or softening of at least a portion of the second polyester. In some embodiments, the absorbent pad and non-stick film may be heated ultrasonically. For example, lamination of any of the one or more film, or any portion thereof, on any of the one or more absorbent pads, or a portion thereof, can be effected by utilizing ultrasonic energy at frequency that sufficiently promotes thermal bonding by melting or softening of at least a portion of the polymeric material. The ultrasonic frequency can be at least about 20 kHz, such as in a range of from about 20 kHz to 100 kHz, and is typically at about 20 kHz or at about 70 kHz. In accordance with some embodiments of the invention, the absorbent pad and non-stick film may be heated by using gamma radiation. The peel strength between the at least a portion of the one or more non-stick films and the one or more absorbent pads, for 2.54 cm (1 inch) wide samples, can be in a range of from 39.37g/ cm (100 g/inch) to 393,70 g/cm (1,000 g/inch) as determined by peeling apart the film from the pad at a separation rate of about 30,48 cm (12 inches) per minute, at 25°C and 65% humidity.

The dressings and bandages described herein may further include a second film, such as one positioned between the backing substrate and the absorbent pad. In such embodiments, the first non-stick film can be affixed to a bottom side of the absorbent pad and the second film can be affixed to the backing substrate by, for example, utilizing an adhesive. In such embodiments, the additional film can increase the ability of the dressing or bandage to store more wound exudates without increasing the likelihood of the dressing or bandage to adhere to the wound. Any of the second film or additional film can be secured to the absorbent pad or any other component or substrate of the dressing or bandage by utilizing the same technique that would be utilized to secure the one or more non-stick films.

In accordance with some embodiments of the invention, any of the films may further comprise fibers and/or additional reinforcement components. Some non-limiting examples of techniques that may be utilized to fabricate the various films include extruding, spraying, casting, and molding.

The backing material or substrate may be made from any suitable material for covering wounds. Non-limiting examples of the backing substrate include polymeric films, paper substrates, woven fabrics, and non-woven fabrics. Examples of polymeric films as well as techniques for making such films that can be used as backing substrates are disclosed in, for example, U.S. Patent No. 6,326,081 B1. In accordance with some embodiments of the invention, the backing substrate may be made from at least one thermoplastic polymer. In accordance with some embodiments of the invention, the backing substrate may be made from at least one thermosetting polymer. Suitable thermoplastic polymers may include, but are not limited to, polyolefins, such as low density polyethylene and linear low density polyethylene, polyvinyl alcohol, nylon, polyester, polystyrene, polymethylpentene, polyoxymethylene, and combinations or mixtures thereof. In one or more embodiments of the invention, the backing substrate may include an elastic polymer. Suitable elastic polymers include, but are not limited to, metallocene catalyzed copolymers of ethylene with a co-monomer selected from the group consisting of octene, hexene, and butene; polymers from ethylene propylene diene monomer; styrene polymers, such as, but not limited to, styrene-butadiene-styrene and styrene-ethylenebutylene-styrene copolymers; ethylene methyl acrylate copolymers; ethylene vinyl acetate polymers, and mixtures thereof. A mixture of any of thermoplastic, thermosetting, and elastic polymers may also be used.

The backing substrate described herein may be formed using any suitable method. For example, the backing substrate may be extruded, molded, solvent cast, woven, or braided. In other configurations, the backing layer or substrate can be a non-woven component.

In some embodiments of the invention, the backing substrate, or at least a portion thereof, may be perforated or have perforations to provide, for example, fluid permeability therethrough, for example, from one surface to an opposite surface. In some embodiments of the invention, the backing substrate may have one or more embossed side. For example, one side or surface of the backing substrate may be smooth and the other side or surface has a matte finish.

One or more absorbent pads may be utilized in one or more embodiments disclosed herein. The one or more absorbent pads may protect the wound from contamination by dirt and may collect blood, wound exudates, and other fluids. Any one or more of the absorbent pads may be made from natural materials, synthetic materials, or combinations thereof including, for example, natural fibers, such as, but not limited to, cotton and wood pulp fibers, and synthetic fibers, such as, but not limited to, polyester, polyamide, and polyolefin fibers. In some particular configurations, the one or more absorbent pads can comprise, consist essentially of, or consist of cotton. In other particular embodiments of the invention, the one or more absorbent pad comprises, consists essentially of, or consists of rayon fibers. Synthetic fibers comprising two or more polymers may be used. Blends of various kinds or types of fibers may be used in any one or more embodiments of the invention. For example, the one or more absorbent pads may be made from a blend of fibers including a first set of fibers made of viscose and a second set of fibers made from a polyester. Further, any of the fibers may be bicomponent fibers. For example, any one or more of the fibers may have a core comprised of one material, such as a first polymer, and a sheath, on at least a portion of the core, comprised of a second material, such as a second or different polymer. Any of the fibers can have any suitable denier for, for example, collecting a desired amount of fluid. For example, the linear mass density of any of the one or more fibers of the at least one absorbent pad can be in a range of from about 1 to 100 denier.

The one or more absorbent pads utilized in the various dressings and bandages can have a porosity that is suitable for allowing the pad to collect and retain fluids. It is envisioned that any portion of the pad may be porous. Any one or more of the absorbent pads may have uniform porosity, a random degree of porosity, or may have a porosity gradient, which, in some cases, may facilitate fluid management, such as fluid flow, in the absorbent pad. The pores may of any of the one or more absorbent pads may be created using any suitable method. For example, any of the one or more absorbent pads may include woven, non-woven fibers, or blends thereof, wherein the space between fibers creates the porosity. In accordance with further embodiments of the invention, any of the one or more absorbent pads may comprise a foam material. For example, any one of the materials described herein may be placed into a solution and lyophilized or freeze-dried to form a foam.

In accordance with some embodiments of the invention, any of the one or more absorbent pads can have a plurality characteristics or properties. For example, the absorbent pad can have at least two regions or portions, such as layers, with differing properties. Non-limiting examples of the various differing properties include density, porosity, diffusivity, and absorbency or absorptive capacity. In one or more embodiments of the invention, the absorbent pad can comprise, consist essentially of, or consist of two or more regions, such as layers, of woven fibers. In one or more embodiments of the invention, the absorbent pad can comprise, consist essentially of, or consist of two or more regions, such as layers, of woven fabrics. In one or more embodiments of the invention, the absorbent pad can comprise, consist essentially of, or consist of two or more regions, such as layers, of non-woven fibers. In one or more embodiments of the invention, the absorbent pad can comprise, consist essentially of, or consist of a first region or layer of woven fibers and a second region or layer of non-woven fibers. In one or more embodiments of the invention, the absorbent pad can comprise, consist essentially of, or consist of a first region or layer with a low density material, and a second region or layer of a high density material. In one or more embodiments of the invention, the absorbent pad can comprise, consist essentially of, or consist of a first, absorbent region or layer comprising a low density fabric, and a second, wicking region or layer comprising a high density fabric. In one or more embodiments of the invention, the absorbent pad can comprise, consist essentially of, or consist of an absorbent region or layer of woven fibers, non-woven fibers, or both. In one or more embodiments of the invention, the absorbent pad can comprise, consist essentially of, or consist of wicking region or layer of woven fibers, non-woven fibers, or both. The absorbent region or layer typically has a greater fluid absorptive capacity than the wicking region or layer. The wicking region or layer typically has a higher fluid diffusivity rate than the diffusivity rate of the absorbent region or layer.

In some cases, the absorbent pad can further comprise, or consist of a third region, such as a layer, comprising, consisting essentially of, or consisting of any of woven fibers, non-woven fibers, or combinations thereof. In some embodiments of the invention, the third region or layer can have at least one characteristic that is similar to any one of the absorbent region or layer and the wicking region or layer. For example, the third region can have the same or about the same absorptive capacity as the absorbent region; or the third region can have the same or about the same fluid diffusivity as the wicking region. In some embodiments of the invention, the third region can have one or more characteristics that are between the characteristics of the first region and the second region. The third region can be proximate any of the first and second regions, and can be disposed between the first and second region. In some configurations, the third region can be disposed at a position that is distal from the first region, thereby disposing the second layer between the first and the third region. For example, the absorbent pad can comprise, consist essentially of, or consist of a first, absorbent region, a second, wicking region disposed against a first side or surface of the absorbent region, and a third, wicking region disposed against a second side or surface of the absorbent region, distal or opposite from the first side or surface.

The absorbent pad can be tailored to provide one or more aggregate properties or characteristics. For example, any of the relative amounts of each of the layers of the absorbent pad can be varied, In accordance with some embodiments of the invention, the ratio of the thickness of one layer to another layer of the absorbent pad can be in a range of from about 1:1 to about 7:1. For example, the ratio of the thickness or relative amount of the absorbent layer to the thickness or relative amount of the wicking layer can be in a range of from about 3:1 to about 5:1. In some particular embodiments of the invention, the ratio of the thickness or relative amount of the absorbent layer to the thickness or relative amount of the wicking layer can be in a range of from about 2:1 to about 4:1, in some cases, in a range of from about 2.5:1 to about 3.5:1.

Any of the absorbent pads can have any desired basis weight and is typically in a range of from, for example, 0.001 g/cm² to 0.25 g/cm². Further, the size of any of the absorbent pads may vary depending on several considerations including, for example, the size of the dressing or bandage, and the size of the wound to be protected or treated. In any of the various embodiments of the invention, at least a portion of the absorbent pad can have a density of in a range of from 0.1 to 0.3 g/cm³. Further, in any of the various embodiments of the invention, the at least a portion of the absorbent pad can have a density in a range of less than about 0.1 g/cm³. In some configurations some embodiments of the invention, the absorbent layer can have a density in a range of from 0.01 g/cm³ to 0.05 g/cm³. The absorbent pad can have 101,71 grams per square meter (3 ounces per square yard) to 339.057 grams per square meter (10 ounces per square yard) of surface area. In some configurations of some embodiments of the invention, the absorbent pad can have about 237,34 grams per square meter (7 ounces per square yard) of surface area.

The absorbent pad may include at least one therapeutic agent. A therapeutic agent is a compound which may provide a therapeutic or prophylactic effect. Some suitable examples of the at least one therapeutic agent include compounds that effect or participate in any one or more of tissue growth, cell growth, and cell differentiation. Further, the at least one therapeutic agent can be an anti-adhesive compound, or a compound that may invoke a biological action, such as an immune response, or participate in one or more biological processes. The therapeutic agent may be applied to and/or incorporated into the pad, or any other part of the dressing or adhesive bandage described herein in any suitable form, e.g., films, powders, liquids, and gels.

Examples of classes of therapeutic agents, which may be utilized in accordance with the present disclosure include anti-adhesives, antibacterial agents, antimicrobial agents, antibiotic agents, antifungal agents, antiviral agents, anticoagulants, anticonvulsants, antidepressants, analgesics, antipyretics, anesthetics, antiepileptics, antihistamines, anti-inflammatories, cardiovascular drugs, diagnostic agents, sympathomimetics, cholinomimetics, antimuscarinics, antispasmodics, hormones, growth factors, muscle relaxants, adrenergic neuron blockers, antineoplastics, immunogenic agents, immunological agents, immunosuppressants, gastrointestinal drugs, diuretics, steroids, lipids, lipopolysaccharides, polysaccharides, platelet activating drugs, clotting factors, and enzymes. It is also intended that combinations of therapeutic agents may be used.

Suitable antimicrobial agents, antifungal agents, antiseptic agents, or antibiotic agents which may be included as the at least one therapeutic agent include, for example, polyhexamethylene biguanides (PHMB); triclosan or 2,4,4'-trichloro-2'-hydroxydiphenyl ether; chlorhexidine and salts thereof, including chlorhexidine acetate, chlorhexidine gluconate, chlorhexidine hydrochloride, and chlorhexidine sulfate; silver and salts or complexes thereof, including silver acetate, silver benzoate, silver carbonate, silver citrate, silver iodate, silver iodide, silver lactate, silver laurate, silver nitrate, silver oxide, silver palmitate, silver protein, and silver sulfadiazine; polymyxin, tetracycline; aminoglycosidic antibiotics, such as, but not limited to, tobramycin and gentamicin; rifampicin; bacitracin; neomycin; chloramphenicol; miconazole; fluoroquinolone and quinolone based agents such as oxolinic acid, norfloxacin, nalidixic acid, pefloxacin, enoxacin and ciprofloxacin; penicillins such as oxacillin and pipracil; nonoxynols; fusidic acid, cephalosporins; and combinations thereof. In addition, antimicrobial proteins and peptides such as bovine lactoferrin and lactoferricin B may be utilized as the at least one therapeutic agent. In accordance with some particular embodiments, any of the dressings or adhesive bandages described herein includes polyhexamethylene biguanide.

Other therapeutic agents can be local anesthetics; non-steroidal antifertility agents; parasympathomimetic agents; psychotherapeutic agents; tranquilizers; decongestants; sedative hypnotics; steroids; sulfonamides; sympathomimetic agents; vaccines; vitamins; antimalarials; anti-migraine agents; anti-parkinson agents such as L-dopa; anti-spasmodics; anticholinergic agents such as oxybutynin; cough suppressants or antitussive agents; bronchodilators; cardiovascular agents such as coronary vasodilators and nitroglycerin; alkaloids; analgesics; narcotics such as codeine, dihydrocodeinone, meperidine, and morphine; non-narcotic agents such as salicylates, aspirin, acetaminophen, and d-propoxyphene; opioid receptor antagonists such as naltrexone and naloxone; anti-cancer agents; anti-convulsants; anti-emetics; antihistamines; antiinflammatory agents such as hormonal agents, hydrocortisone, prednisolone, prednisone, non-hormonal agents, allopurinol; indomethacin; phenylbutazon; prostaglandins and cytotoxic drugs; chemotherapeutic agents; and estrogens.

Other examples of suitable therapeutic agents, which may be included in any one or more of the backing layer or substrate, the absorbent pad, the adhesive layer, and the non-stick film include, viruses and cells; peptides, polypeptides and proteins, as well as analogs, muteins, and active fragments thereof; immunoglobulins; antibodies; cytokines such as lymphokines, monokines, and chemokines; blood clotting factors; hemopoietic factors; interleukins such as IL-2, IL-3, IL-4, and IL-6; interferons such as β-IFN, α-IFN and γ-IFN; erythropoietin; nucleases; tumor necrosis factor; colony stimulating factors such as GCSF, GM-CSF, and MCSF; insulin; anti-tumor agents and tumor suppressors; blood proteins such as fibrin, thrombin, fibrinogen, synthetic thrombin, synthetic fibrin, synthetic fibrinogen; gonadotropins such as FSH, LH, and CG; hormones and hormone analogs such as growth hormone; vaccines such as tumoral, bacterial and viral antigens; somatostatin; antigens; blood coagulation factors; growth factors such as nerve growth factor and insulin-like growth factor; bone morphogenic proteins; TGF-B; protein inhibitors; protein antagonists; protein agonists; nucleic acids, such as antisense molecules, DNA, RNA, RNAi; oligonucleotides; polynucleotides; and ribozymes.

Anti-adhesive agents can be used to prevent adhesions from forming between the bandage and the wound or tissue surrounding the wound. Some examples of these agents include, but are not limited to, hydrophilic polymers such as poly(vinyl pyrrolidone), carboxymethyl cellulose, hyaluronic acid, polyethylene oxide, polyvinyl alcohols, and combinations thereof.

An adhesive material or layer may be used to adhere the absorbent pad to the backing material and, in some cases, to secure the dressing or adhesive bandage to the skin of the user. The adhesive may be an aqueous solvent-based compound. In other configurations, the adhesive may be a non-aqueous solvent-based compound. In some cases, the adhesive material may comprise amorphous polyolefins including amorphous polypropylene, ethylene copolymers such as ethylene vinyl acetate copolymers, and combinations thereof. Suitable adhesives also include acrylic based, dextrin based, and urethane based adhesives as well as natural and synthetic elastomers, and blends thereof. In still other cases, the adhesive material may be a hot melt adhesive. Examples of suitable adhesive materials include, but are not limited to, those based on styrenic block copolymers and tackifying resins. The adhesives may also include any one or more of tackifiers, anti-oxidants, and processing oils. Sources of commercially available adhesive components include Kraton Performance Polymers Inc., Houston, Texas; H.B. Fuller Company, St. Paul, Minnesota; Bostik Inc., Wauwatosa, Wisconsin; National Starch, Bridgewater, New Jersey; and REXtac, LLC, Odessa, Texas.

The adhesive can be applied in any desired manner such as by spraying, screen printing, or slot die coating to suitable amount. The adhesive coating weight can vary from about 20 grams per square meter (gsm) to about 100 gsm. In embodiments of the invention, the adhesive may be applied to a permeable backing layer at an amount that maintains the permeability of the backing layer or substrate. For example, a backing layer comprised of woven or non-woven fibers may have a desired porosity between the fibers prior to the application of the adhesive and after the application of the adhesive. In other embodiments of the invention, the adhesive may be applied in an amount that renders the porous backing layer impermeable to fluids.

Any of the dressings and bandages as disclosed herein may have any geometrical configuration and any suitable dimension. For example, the dressing or bandages can be shaped to be square, rectangular, round, oval, or triangular. The size of the dressing or bandage will depend on the shape of the dressing or bandage and the size of the wound meant to be covered by the dressing or bandage. A square bandage may range in size from about 2 cm X 2 cm to about 15 cm X 15 cm. A rectangular bandage may range in size from about 5 cm to about 15 cm, in some cases, from about 7.5 cm to about 12.5 cm and the width of the rectangular bandage may range from about 0.5 cm to 5 cm, in some cases, from about 1 cm to about 3 cm.

The dressing or bandage can have any suitable thickness, depending on the application or use. Typically, the thickness of the dressing or bandage can range from about 0.25 mm to about 5 mm; in some embodiments of the invention, the thickness can range from about 1 mm to about 3 mm; and in some further embodiments of the invention, the thickness can range from about 1 mm to about 2 mm.

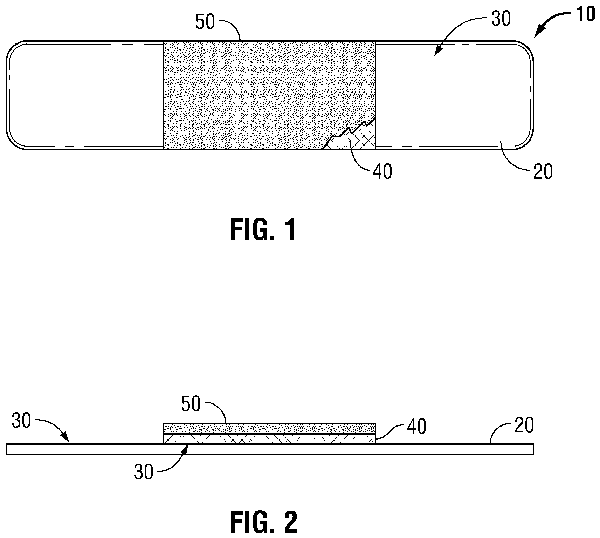

FIGS. 1 and 2 exemplarily illustrate a dressing in accordance with some aspects of the invention. Adhesive bandage 10 can comprise a backing substrate 20 affixed to an absorbent pad 40 via an adhesive 30. Adhesive 30 is shown positioned along the entire length of backing substrate 20; however it envisioned that adhesive 30 may be positioned along any portion of backing substrate suitable, such as for adhering the dressing as an adhesive bandage to skin or tissue. Non-stick film 50 comprises a first polyester and a second polyester having a lower melting point than the first polyester. Non-stick film 50 can be affixed to the wound-facing side or surface of absorbent pad 40. Non-stick film 50 may be a film which includes a blend of a polyester and an amorphous polyester wherein the amorphous polyester displays a lower melting point than the first polyester material.

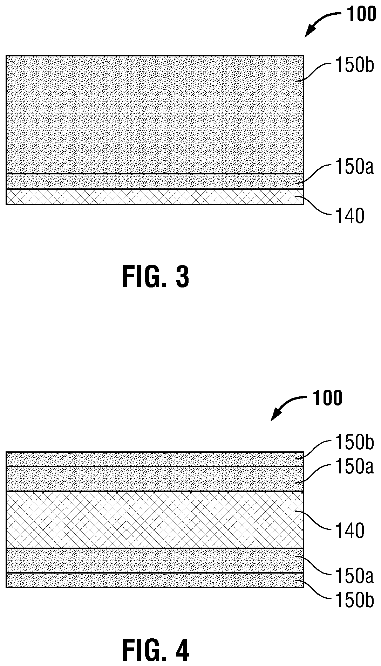

As exemplarily illustrated in FIGS. 3 and 4, a dressing 100 can comprise an absorbent pad 140, which can be optionally secured to an backing substrate (not shown) via an adhesive. Absorbent pad 140 can be positioned to be generally centered along the entire length of the backing substrate or along any portion thereof. The non-stick film can comprise a first and a second polyester layer. As exemplarily illustrated, second polyester layer 150a can be affixed to the wound-facing surface of absorbent pad 140 and a first polyester layer 150b of the non-stick film can be attached to the second polyester layer 150a. The polyester of second polymeric layer 150a has a lower melting point than the polyester of the first polymeric layer 150b. The second polymeric layer consists essentially of an amorphous polyester and the first polymeric layer consists essentially of a different polyester, such as an at least partially crystalline polyester.

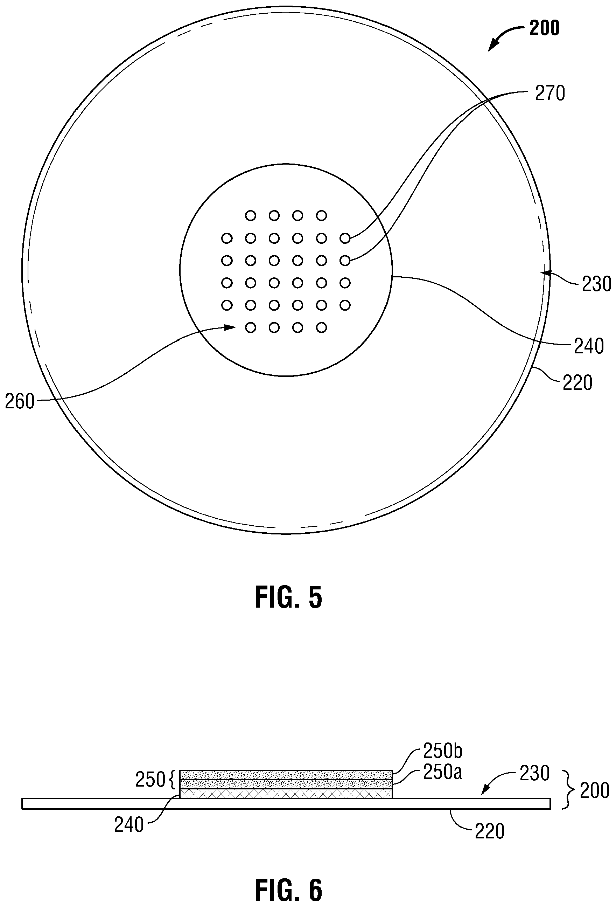

The dressing or bandages of the present invention may be of any suitable shape or size for covering a wound. As exemplarily illustrated in FIGS. 5 and 6, adhesive bandage 200 may be an island dressing and/or circular configuration with absorbent pad 240 affixed to a generally central portion of backing substrate 220 via, for example, an adhesive 230. As shown in FIG. 6, the non-stick film 250 can have a first layer 250b that is secured or affixed to absorbent pad 240 via a second layer 250a of the non-stick film at about around an outer perimeter of absorbent pad 240. Second layer 250a typically has a lower melting point than the melting point of the first polymeric layer 250b. In such an embodiment, first non-stick film 250a may create an opening or channel between second layer 250b and absorbent pad 240 to allow wound exudates to flow through during the wound healing process. Because the second polymeric layer 250a is advantageously positioned along the outer perimeter of the absorbent pad 240, the second polymeric layer 250a would likely not interfere with the absorption of wound exudates or other fluid through the central portion of absorbent pad 240. The non-stick film can have one or more perforated regions 260, each of which can have a plurality perforations or apertures 270 through the thickness of the film.

The dressings and bandages of the present invention can fabricated by providing a strip of backing substrate having the desired dimensions, and optionally applying an adhesive thereon. An absorbent pad is typically provided. Optionally, the absorbent pad may be adhered to a desired region of the backing substrate. A non-stick film may be secured to the absorbent pad by at least partially melting or softening a polymeric layer of the non-stick film, positioning the at least partially melted or softened non-stick film on the absorbent pad; and optionally applying pressure to disposed at least a portion of the melted or softened polymeric matrix in the absorbent pad. Release strips, such as siliconized paper, can optionally be placed over the exposed portions of adhesive-applied region as well as on the non-stick film. The bandage is then packaged by, for example, enclosing it between two layers of heat sealable paper, and heat sealing the periphery of the two paper layers. The packaged bandage can then be sterilized, if desired. When utilized, the absorbent pad, the non-stick film, or both can be immersed in a bath comprising the one or more therapeutic agent. The wet pads may be allowed to dry.

The absorbent pad may be made from a non-woven blend of fibers including viscose fibers, representing 80% by weight of the pad and a polyester fiber representing 20% by weight of the pad. The absorbent pad may initially be formed into long strips or rolls suitable for being cut into a multitude of smaller absorbent pads. The long strips or rolls of pads may then be passed through a solution containing a therapeutic agent, such as an antimicrobial agent like PHMB.

The composite assembly comprising the absorbent pad and the non-stick film may then be cut into a desired dimension.

It will be understood that various modifications may be made to the embodiments disclosed herein. For example, the adhesive bandage may be an absorbable bandage. In another example, the adhesive bandages described herein may be sterilized using any suitable sterilization process, such as gamma radiation, and packaged into any suitable medical device package, such as an injectable medical device package. Those skilled in the art should appreciate that the parameters and configurations described herein are exemplary and that actual parameters and/or configurations will depend on the specific application in which the systems and techniques of the invention are used. For example, a unitary non-stick film may be utilized to wrap around any of the absorbent pads described herein, with the non-stick film having a plurality of polymeric regions or layers with differing melting points, melting ranges or softening points, and with the absorbent pad having one or more layers or regions, each of which can have differing characteristics, and wherein each of the various polymeric layers of the non-stick film is a polyester with differing melting ranges and each of the layers of the absorbent pad utilizes 100% cotton fibers.

As used herein, the term "plurality" refers to two or more items or components. Use of ordinal terms, such as "first," "second," "third," in the claims to modify a claim element does not by itself connote any priority, precedence, or order of one claim element over another or the temporal order in which acts of a method are performed, but are used merely as labels to distinguish one claim element having a certain name from another element having a same name, but for use of the ordinal term, to distinguish the claim elements. The terms "comprising," "including," "carrying," "having," "containing," and "involving," whether in the written description or the claims, are open-ended terms. Thus, the use of such terms is meant to encompass the items listed thereafter, and equivalents thereof, as well as additional items. Only the transitional phrases "consisting of" and "consisting essentially of," are closed or semi-closed transitional phrases, respectively, with respect to the claims.

## Claims

1. A dressing (100,200) for applying on a wound, comprising:
an absorbent pad (140,240) having a wound-facing surface and a second surface opposite from the wound-facing surface, wherein the absorbent pad (140,240) consists essentially of cellulosic fibers and polymeric fibers; and
a non-stick film (250) secured to the absorbent pad (140,240) on the wound-facing surface, the non-stick film (250) having a first region (150b,250b) consisting essentially of a first polyester and a second region (150a,250a) consisting essentially of a second polyester having a lower melting point than the first polyester.

2. The dressing (100,200) of claim 1, wherein the second region defines a securing layer attaching the non-stick film to the wound-facing surface of the absorbent pad.

3. The dressing (100,200) of any one of claims 1 or 2, wherein the second polyester comprises an amorphous polyester having a melting temperature range in a range of from 120°C to 200°C and wherein the first polyester comprises polyethylene terephthalate having a melting temperature range in a range of from 200°C to 275°C.

4. The dressing (100,200) of any one of the preceding claims, wherein the second polyester comprises polyethylene terephthalate having a glass transition temperature in a range of from 60°C to 88°C.

5. The dressing (100,200) of any one of the preceding claims, further comprising a second film secured to the absorbent pad on the second surface thereof, the second film having a third region comprising the first polymeric material and a fourth region comprising the second polymeric material, and wherein the fourth region defines a second securing layer attaching the second film to the second surface of the absorbent pad.

6. The dressing (100,200) of any one of the preceding claims, further comprising a backing substrate having an adhesive material on a surface thereof.

7. The dressing (100,200) of claim 6, wherein the backing substrate comprises a woven fabric and the adhesive material comprises an acrylic.

8. The dressing (100,200) of any one of the preceding claims, wherein the non-stick film (250) has a perforated region (260) including a plurality of perforations (270) extending through the thickness thereof, and wherein the cellulosic fibers are selected from the group consisting of viscose, cotton, fiberized paper pulp, lyocell, and combinations thereof, and the polymeric fibers are selected from the group consisting of polypropylene fibers, polyester fibers, and combinations thereof.

9. The dressing (100,200) of any one of the preceding claims, wherein the absorbent pad (140,240) has a first, absorbent layer comprising a low density absorbent fabric and a second, wicking layer comprising a high density absorbent fabric.

10. The dressing (100,200) of any one of the preceeding claims, wherein the absorbent pad (140,240) comprises polyhexamethylene biguanide.

11. A method of producing a wound dressing (100,200), comprising:
securing a non-stick film (250) to an absorbent pad (140,240) at a wound-facing surface thereof, wherein the non-stick film comprises a first region (150b, 250b) consisting essentially of a first polyester and a second region (150a, 250a) consisting essentially of a second polyester, the second polyester having a lower melting point than the first polyester, and wherein the absorbent pad (140,240) consists essentially of cellulosic fibers and polymeric fibers.

12. The method of claim 11, wherein the first polyester has a melting range in a range of from 200°C to 275°C, and the second polyester has a melting range in a range of from 120°C to 200°C.

13. The method of any one of claims 11 to 12, wherein securing the non-stick film (250) to the absorbent pad (140,240) comprises exposing the non-stick film (250) to an environment with a temperature in a range of from 140°C to 200°C.

14. The method of any one of claims 11 to 13, further comprising:
immersing at least a portion of the absorbent pad (140,240) in a fluid comprising polyhexamethylene biguanide to produce a PHMB-immersed pad; and
drying the PHMB-immersed pad to produce a PHMB- containing pad, and
wherein securing the non-stick film (250) to the absorbent pad (140) comprises securing the second region of the non-stick film (250) to the PHMB-containing pad.

15. The dressing (100,200) of claim 1, wherein the absorbent pad (140,240) consists essentially of a blend of 80 wt% viscose fibers and 20 wt% polyester fibers.

16. The method (100,200) of claim 11, wherein the absorbent pad (140,240) consists essentially of a blend of 80 wt% viscose fibers and 20 wt% polyester fibers.

## Patentansprüche

1. Verband (100, 200) zum Aufbringen auf eine Wunde, umfassend:
eine absorptionsfähige Kompresse (140, 240), die eine der Wunde zugewandte Oberfläche und eine zweite Oberfläche, die der der Wunde zugewandten Oberfläche entgegengesetzt ist, aufweist, wobei die absorptionsfähige Kompresse (140, 240) im Wesentlichen aus cellulosischen Fasern und polymeren Fasern besteht; und
eine nichtklebrige Folie (250), die an der absorptionsfähigen Kompresse (140, 240) auf der der Wunde zugewandten Seite befestigt ist, wobei die nichtklebrige Folie (250) eine erste Region (150b, 250b), die im Wesentlichen aus einem ersten Polyester besteht, und eine zweiten Region (150a, 250a) aufweist, die im Wesentlichen aus einem zweiten Polyester besteht, der einen niedrigeren Schmelzpunkt als der erste Polyester aufweist.

2. Verband (100, 200) nach Anspruch 1, wobei die zweite Region eine Befestigungsschicht definiert, die die nichtklebrige Folie an die der Wunde zugewandten Oberfläche der absorptionsfähigen Kompresse anbringt.

3. Verband (100, 200) nach irgendeinem der Ansprüche 1 oder 2, wobei der zweite Polyester einen amorphen Polyester umfasst, der einen Schmelztemperaturbereich in einem Bereich von 120°C bis 200°C aufweist und wobei der erste Polyester Polyethylenterephthalat umfasst, das einen Schmelztemperaturbereich von 200°C bis 275°C aufweist.

4. Verband (100, 200) nach irgendeinem der vorhergehenden Ansprüche, wobei der zweite Polyester Polyethylenterephthalat umfasst, das eine Glasübergangstemperatur in einem Bereich von 60°C bis 88°C aufweist.

5. Verband (100, 200) nach irgendeinem der vorhergehenden Ansprüche, ferner eine zweite Folie umfassend, die an der absorptionsfähigen Kompresse auf der zweiten Oberfläche davon befestigt ist, wobei die zweite Folie eine dritte Region, die das erste polymere Material umfasst, und eine vierte Region aufweist, die das zweite polymere Material umfasst und wobei die vierte Region eine zweite Befestigungsschicht definiert, die die zweite Folie an die zweite Oberfläche der absorptionsfähigen Kompresses anbringt.

6. Verband (100, 200) nach irgendeinem der vorhergehenden Ansprüche, ferner ein Trägerstoffsubstrat umfassend, dass ein Klebstoffmaterial auf einer Oberfläche davon aufweist.

7. Verband (100, 200) nach Anspruch 6, wobei das Trägerstoffsubstrat einen gewobenen Textilstoff umfasst und das Klebstoffmaterial ein Acryl umfasst.

8. Verband (100, 200) nach irgendeinem der vorhergehenden Ansprüche, wobei die nichtklebende Folie (250) eine durchlöcherte Region (260) aufweist, die eine Mehrzahl von Perforationen (270) umfasst, die sich durch die Dicke davon erstrecken, und wobei die cellulosischen Fasern aus der Gruppe ausgewählt sind bestehend aus Viskose, Baumwolle, faserisierter Papierpulpe, Lyocell und Kombinationen davon und die polymeren Fasern aus der Gruppe ausgewählt sind bestehend aus Polypropylenfasern, Polyesterfasern und Kombinationen davon.

9. Verband (100, 200) nach irgendeinem der vorhergehenden Ansprüche, wobei die absorptionsfähige Kompresse (140, 240) eine erste, absorptionsfähige Schicht, die einen absorptionsfähige Textilstoff geringer Dichte umfasst, und eine zweite Dochtstoffschicht, die einen absorptionsfähige Textilstoff hoher Dichte umfasst aufweist.

10. Verband (100, 200) nach irgendeinem der vorhergehenden Ansprüche, wobei die absorptionsfähige Kompresse (140, 240) Polyhexamethylenbiguanid umfasst.

11. Verfahren für die Herstellung eines Wundverbands (100, 200), umfassend:
Befestigen einer nichtklebrigen Folie (250) an einer absorptionsfähigen Kompresse (140, 240) auf einer der Wunde zugewandten Seite, wobei die nichtklebrige Folie eine erste Region (150b, 250b), die im Wesentlichen aus einem ersten Polyester besteht, und eine zweiten Region (150a, 250a) umfasst, die im Wesentlichen aus einem zweiten Polyester besteht, wobei der zweite Polyester einen niedrigeren Schmelzpunkt als der erste Polyester aufweist und wobei das absorptionsfähige Kissen (140, 240) im Wesentlichen aus cellulosischen Fasern und polymeren Fasern besteht.

12. Verfahren nach Anspruch 11, wobei der erste Polyester einen Schmelzbereich in einem Bereich von 200°C bis 275°C aufweist und der zweite Polyester einen Schmelzbereich in einem Bereich von 120°C bis 200°C aufweist.

13. Verfahren nach irgendeinem der Ansprüche 11 bis 12, wobei das Befestigen der nichtklebrigen Folie (250) an der absorptionsfähigen Kompresse (140, 240) das Aussetzen der nichtklebrigen Folie (250) einer Umgebung mit einer Temperatur in einem Bereich von 140°C bis 200°C umfasst.

14. Verfahren nach irgendeinem der Ansprüche 11 bis 13, ferner Folgendes umfassend:
Eintauchen mindestens eines Teils der absorptionsfähigen Kompresses (140, 240) in ein Fluid, das Polyhexamethylenbiguanid umfasst, um eine in PHMB eingetauchte Kompresse herzustellen; und
Trocknen der in PHMB eingetauchten Kompresse, um eine PHMP enthaltende Kompresse herzustellen, und
wobei das Befestigen der nichtklebrigen Folie (250) an der absorptionsfähigen Kompresse (140) das Befestigen der zweiten Region der nichtklebrigen Folie (250) an der PHMP enthaltenden Kompresse umfasst.

15. Verband (100, 200) nach Anspruch 1, wobei die absorptionsfähige Kompresse (140, 240) im Wesentlichen aus einer Mischung von 80 Gew.-% Viskosefasern und 20 Gew.-% Polyesterfasern besteht.

16. Verfahren (100, 200) nach Anspruch 11, wobei die absorptionsfähige Kompresse (140, 240) im Wesentlichen aus einer Mischung von 80 Gew.-% Viskosefasern und 20 Gew.-% Polyesterfasern besteht.

## Revendications

1. Pansement (100, 200) à appliquer sur une plaie, comprenant:
un tampon absorbant (140, 240) ayant une surface faisant face à la plaie et une seconde surface opposée à la surface faisant face à la plaie, le tampon absorbant (140, 240) étant essentiellement constitué de fibres cellulosiques et de fibres polymères; et
un film antiadhésif (250) solidement fixé au tampon absorbant (140, 240) sur la surface faisant face à la plaie, le film antiadhésif (250) ayant une première région (150b, 250b) constituée essentiellement d'un premier polyester et une seconde région (150a, 250a) constituée essentiellement d'un second polyester ayant un point de fusion inférieur au premier polyester.

2. Pansement (100, 200) selon la revendication 1, la seconde région définissant une couche de fixation solide fixant le film antiadhésif à la surface faisant face à la plaie du tampon absorbant.

3. Pansement (100, 200) selon l'une quelconque des revendications 1 ou 2, le second polyester comprenant un polyester amorphe ayant une plage de températures de fusion située dans une plage de 120°C à 200°C et le premier polyester comprenant du poly(téréphtalate d'éthylène) ayant une plage de températures de fusion située dans une plage de 200°C à 275°C.

4. Pansement (100, 200) selon l'une quelconque des revendications précédentes, le second polyester comprenant du poly(téréphtalate d'éthylène) ayant une température de transition vitreuse située dans une plage de 60°C à 88°C.

5. Pansement (100, 200) selon l'une quelconque des revendications précédentes, comprenant en outre un second film solidement fixé au tampon absorbant sur sa seconde surface, le second film ayant une troisième région comprenant le premier matériau polymère et une quatrième région comprenant le second matériau polymère, et la quatrième région définissant une seconde couche de fixation solide fixant le second film à la seconde surface du tampon absorbant.

6. Pansement (100, 200) selon l'une quelconque des revendications précédentes, comprenant en outre un substrat dorsal ayant un matériau adhésif sur une surface de celui-ci.

7. Pansement (100, 200) selon la revendication 6, le substrat dorsal comprenant un tissu tissé et le matériau adhésif comprenant un acrylique.

8. Pansement (100, 200) selon l'une quelconque des revendications précédentes, le film antiadhésif (250) ayant une région perforée (260) comprenant une pluralité de perforations (270) s'étendant à travers son épaisseur, et les fibres cellulosiques étant sélectionnées parmi le groupe constitué de la viscose, du coton, de la pâte de papier défibré, de la Lyocell, et de leurs combinaisons, et les fibres polymères étant sélectionnées dans le groupe constitué des fibres de polypropylène, des fibres de polyester, et de leurs combinaisons.

9. Pansement (100, 200) selon l'une quelconque des revendications précédentes, le tampon absorbant (140, 240) ayant une première, couche absorbante comprenant un tissu absorbant de faible densité et une seconde, couche d'imbibition comprenant un tissu absorbant de haute densité.

10. Pansement (100, 200) selon l'une quelconque des revendications précédentes, le tampon absorbant (140, 240) comprenant du biguanide de polyhexaméthylène.

11. Procédé de production d'un pansement pour plaie (100, 200), comprenant:
la fixation solide d'un film antiadhésif (250) à un tampon absorbant (140, 240) à sa surface faisant face à la plaie, le film antiadhésif comprenant une première région (150b, 250b) constituée essentiellement d'un premier polyester et d'une seconde région (150a, 250a) constituée essentiellement d'un second polyester, le second polyester ayant un point de fusion inférieur au premier polyester, et le tampon absorbant (140, 240) étant essentiellement constitué de fibres cellulosiques et de fibres polymères.

12. Procédé selon la revendication 11, le premier polyester ayant une plage de fusion située dans une plage de 200°C à 275°C, et le second polyester ayant une plage de fusion située dans une plage de 120°C à 200°C.

13. Procédé selon l'une quelconque des revendications 11 à 12, la fixation solide du film antiadhésif (250) au tampon absorbant (140, 240) comprenant l'exposition du film antiadhésif (250) à un environnement ayant une température située dans une plage de 140°C à 200°C.

14. Procédé selon l'une quelconque des revendications 11 à 13, comprenant en outre:
l'immersion au moins d'une partie du tampon absorbant (140, 240) dans un liquide comprenant du biguanide de polyhexaméthylène pour produire un tampon immergé dans du PHMB; et
le séchage du tampon immergé dans le PHMB pour produire un tampon contenant du PHMB, et
la fixation solide du film antiadhésif (250) au tampon absorbant (140) comprenant la fixation solide de la seconde région du film antiadhésif (250) au tampon contenant du PHMB.

15. Pansement (100, 200) selon la revendication 1, le tampon absorbant (140, 240) étant essentiellement constitué d'un mélange de 80 % en pds de fibres de viscose et de 20 % en pds de fibres de polyester.

16. Procédé (100, 200) selon la revendication 11, le tampon absorbant (140, 240) étant essentiellement constitué d'un mélange de 80 % en pds de fibres de viscose et de 20 % en pds de fibres de polyester.
